# EUROPEAN PATENT APPLICATION

(11) **EP 2 236 544 A1**
(43) Date of publication of application: **06.10.2010**
(21) Application number: 09155963.3
(22) Date of filing: 24.03.2009
(51) Int. Cl.: C08H 1/06

(54) **Collagen Implant**

(71) Applicant: Friedrich-Alexander-Universität Erlangen-Nürnberg, 91054 Erlangen (DE)
(72) Inventor: Breiter Roman, 91052 Erlangen (DE); Körber Ludwig, 91126 Schwabach (DE); Schwarz Silke, 91126 Schwabach (DE)
(74) Representative: Behnisch, Werner

(57) **Abstract**

The invention relates to a highly pure collagen, its use as an implant and a method for its preparation, the method comprising subjecting a sample comprising collagen to at least one alkaline treatment step in an alkaline solution at a pH > 12 and at least one cleaning step with at least one chaotropic agent.

## Description

The invention relates to a highly pure collagen, its use as an implant methods for its preparation, the method comprising subjecting a sample comprising collagen to at least one alkaline treatment step in an alkaline solution at a pH >12 and at least one cleaning step with at least one chaotropic agent.

### Prior Art

Attempts have been made in the art to prepare collagen for use in implants from natural tissue, wherein the tissue is treated to remove non-collagenous components such as cells, cellular debris and other extracellular matrix components. Such pure implants are important for surgical replacement of non-functional tissue. A high degree of purity is important to avoid an immunogenic rejection of the implant and infections by pathogens. Rejection of implants can be mediated by natural antibodies (anti-Gal) in serum. Chronic rejections can be mediated by inflammatory cells which enter the implant. There is thus a need for a collagen implant from which all cells and antigens have been removed.

Purification methods known in the art frequently do not provide a sufficiently pure collagen. Further, the methods are not efficient for removing impurities from a collagen scaffold when the tissue has a high density.

A tissue of especially high density is meniscus tissue. The material high density confers the necessary mechanical properties to the meniscus, such as pressure stability, pressure conversion, stabilization of the knee joint and shock absorption. Meniscus injuries are some of the most frequent injuries resulting from sports activities and are caused by excessive strain of knee joints. The number of meniscus injuries increases especially in the group of people younger than 30 by new high risk sports. In the group of people older than 50, increased leisure activities result in a continuously rising number of meniscus injuries. In 2004 the number of meniscus injuries in Germany is estimated at 300,000 and in the US, more than 1 million interventions are performed. Only 10 % of all meniscus injuries are remediable by arthroscopic knee surgery, and very often a complete meniscus ectomy is necessary. Within a short time after complete or partial meniscus ectomy, it leads to an increasing joint deterioration, and direct bone-on-bone contact. Patients get pain, swelling, instability of knee joints and early degenerative arthropathies. An adequate meniscus graft is getting more and more important in reconstructive and regenerative medicine, traumatology and orthopedics.

Several meniscus joints are known. Permanent prostheses from synthetic materials, such as Teflon or Dacron, do not show satisfactory results in animal tests. Material failure and synovitis induced by abrasion are observed.

Several autologous tissues have been tested in animal experiments (Goble EM 1999) and clinically as meniscal grafts (Wirth et al. 2002). Diverse conserved allogeneic meniscus tissues, such as irradiated, lyophilised, cryoconserved or fresh donor tissues, have been transplanted. So far no allogeneic graft has been shown to provide protection from degenerative changes of the articular surface. The risk of transmission of infectious diseases, of immune response and incompatibilities is high (Soldner and Herr 2001). Dehydrated grafts may shrink, and the microstructure may be modified. Chronic inflammation, non-specific synovitis and chondro-mucoid degeneration arise (Rodeo et al. 2001).

Resorbable, collageneous scaffold of highly purified collagen (almost exclusively collagen I) with and without crosslinking is only used for partial meniscus repair (e.g. CMI from ReGen Biologics). Extracellular matrices (e.g. Cook from Cook Biotech), prepared from intestinal mucosa, are grouted for use. The resulting collagenous scaffold does not show any ordered structure of collagen fibers as a natural bioimplant.

Xenogeneic grafts are unlimited and readily available, but antigenic and hyperacute rejection reactions can occur, primarily caused by the interaction of natural antibodies with specific alpha-Gal-epitopes.

Therefore there is the need for a collagenous material, which has material properties, biochemical stability and integrity, collagen composition, packing of 3-dimensional collagen, fibrils and fibres to a great extent as in natural tissue. The matrix is the scaffold for immigrating cells, and their differentiation and redifferentiation into chondrogenic cells (Delloye et al. 2007). All components which might cause immune responses and rejection reactions have to be denatured and removed (Goncalves et al. 2005). The risk for transmission of infectious diseases must be minimized. The scaffold should allow the colonization, recellularisation, and proliferation of cells, the diffusion of nutrients and other relevant substances. It should be replaceable by a regenerate matrix. Therefore the matrix should be chondroconductive and chondroinductive after implantation and immigrating bodies cells.

US 5,374,539 discloses a method for producing a collagen composition from collagenous animal tissue, in which the non-collagenous material is removed with a proteolytic enzyme digesting solution. However, the enzyme solution is not capable of removing the non-collagenous components from a collagen matrix, because enzymes do not diffuse efficiently into the matrix. The method thus requires an initial homogenization step, in which the collagen is disrupted, and a final cross-linking step. In general, enzyme preparations, especially those comprising a large number of enzymes, are expensive and their handling is relatively complicated. Care has to be taken that the enzymes are not themselves degraded and maintained within the matrix. The method of US 5,374,539 is not applicable for preparing a highly pure natural collagen matrix from a dense tissue such as meniscus.

A further method for purifying collagen is known from US 5,993,844. According to this method, a native mammalian soft tissue comprising collagen is treated with an alkaline solution containing a chelating agent, an acidic solution containing a salt, a salt solution and a rinse agent. The method is not efficient for preparing a highly pure collagen matrix from a dense tissue, such as meniscus.

In summary, according to the methods known in the art, collagen obtained from tissue is generally not sufficiently pure. Due to the low purity, it is not possible to use the collagen as an implant because of the high immunogenity due to the impurities. In case the collagen is used as an implant, undesirable responses such as inflammation and collagen disintegration are observed. The known methods are especially not applicable for the preparation of collagen, which is in the form of a dense or thick matrix.

The problem underlying the invention is to provide a highly pure collagen and a method for its preparation, which overcome the problems outlined above. The degree of non-collagenous impurities, such as non-collagenous proteins, nucleic acids and cell debris, shall be low, such that the collagen can be used as an implant. Further, pathogens such as viruses and prions shall be removed.

A specific problem underlying the invention is to provide a method, which is efficient for preparing a pure collagen matrix from a dense or thick material. The method shall not require a matrix disintegration step and reassembly of the matrix, for instance by crosslinking or pressing. A specific problem underlying the invention is to provide collagen from a highly dense tissue, and especially from meniscus. The natural structure of the tissue or meniscus collagen scaffold shall be maintained. Further, the method shall be applicable without enzyme digestion steps.

It is a further problem to provide a method for preparing highly pure collagen, which can be carried out relatively simple, with standard chemicals and at low costs, and which can be automated.

### Disclosure of the Invention

Surprisingly, the problem underlying the invention is solved by methods and collagen implants according to the claims.

Subject of the invention is a method for preparing a highly pure collagen, comprising subjecting a sample comprising collagen to
at least one alkaline treatment step in an alkaline solution at a pH >12 and
at least one cleaning step with at least one chaotropic agent.

In general, any natural or artificial tissue comprising collagen can be used. Preferably, the sample is a tissue obtained from an animal. The sample is preferably a tissue of high density and/or thickness. Preferably, the sample comprises a three-dimensional collagen matrix (scaffold). A preferred dense tissue is cartilage. It is preferred that the matrix comprises collagen type I and collagen type II. Preferably, the sample comprises more than 30, 50 or 60 % (by dry weight) collagen.

A preferred sample of the invention is meniscus, which has a compact structure due to a dense collagen matrix. The inventive method allows purification of meniscus whilst preserving the natural scaffold of collagen fibrils. The purified cartilage or meniscus of the invention is porous. It comprises cavities, from which non-collageneous impurities, such as cells, proteoglycans, gylcosaminoglycans and other matrix components, were removed. This is an important prerequisite for diffusion of cells into the matrix after implantation. Only when diffusion throughout the entire matrix occurs after implantation, a cartilage such as meniscus will develop adequate mechanical properties. Since the method of the invention uses harsh chemicals, it cannot be excluded that a small amount of chemical bonds within the collagen matrix are disrupted. However, this is not detrimental to the invention as long as the overall tree-dimensional porous scaffold of the collagen is essentially maintained. A limited extent of disruption of bonds might even be acceptable or advantageous in order to improve the diffusion processes during the purification. In further embodiments, the sample is selected from intervertebral (or spinal) discs, articular discs, ear cartilage, rib cartilage, rib cartilage, nasal cartilage, cruciate ligament and Achilles tendon.

Preferably, the tissue is bovine, porcine or human. Preferably, the collagen has a purity which is so high that it can be used as an implant. An "implant" is a collagen which is usable and/or adapted for transfer to a patient by surgery.

In a preferred embodiment of the invention, the alkaline solution comprises NaOH, KaOH, Ca(OH)₂, CaO, Mg(OH)₂ or LiOH. The pH of the solution is >12, preferably >12.2, >12.5 or >13. The pH can be adjusted with any strong base which may be used in a concentration range between 0.01 and 3 M, preferably between 0.05 M and 2 M. The alkaline treatment is preferably carried out at a temperature between 0 and 40°C, preferably at room temperature. Especially preferred is the use of a NaOH solution. Preferably, the sample is subjected to the alkaline treatment for at least 30 minutes, more preferably for at least one hour or at least two hours. Especially preferred is a treatment of the sample with 1 NaOH for three hours.

It was found that with an alkaline treatment step at a high pH it is possible to hydrolyze in a single step a large amount of contaminants, such as proteins and lipids, without negatively affecting the collagen. Further, the alkaline treatment step allows rapid and simple inactivation of pathogens, such as viruses (hepatitis), retroviruses (PERV), bacteria and prions (BSE, CJD).

The sterilization step is similar to the decontamination of vessels with NaOH used in the art. For instance, it is known in the art that an inactivation of PrP^{Sc} with a reduction factor of >4 log₁₀ is achieved with 0.1 N NaOH solution at room temperature after 15 minutes.

The sample is preferably a tissue of high density and/or thickness. A preferred dense tissue is cartilage. A preferred sample is meniscus tissue, which has a compact structure due to a thick collagen matrix. When the sample is meniscus tissue, it is preferred to apply the alkaline treatment step at least at a pH of 12 or 13 for at least one hour. In general, the thicker the sample structure and the diameter of the sample, the higher should the pH be adjusted and the time of treatment increased. The alkaline concentration should not be adjusted too high, because for instance at a concentration of NaOH of 3 M it was found that meniscus tissue can start disintegration. The purification can be improved if the alkaline treatment step is repeated at least once.

It is preferred to use the alkaline treatment step at the beginning of the inventive procedure, because it was found that the alkaline treatment, for instance with 1 N NaOH, induces rapid and pronounced swelling of the tissue, and consequently reagents in further treatment steps can be applied more efficiently.

In a preferred embodiment, at the end of the alkaline treatment step, the sample is subjected to a sonication. For example, the sonication is applied for 2 to 30 minutes.

After the alkaline treatment, the alkaline solution is removed from the sample. Preferably, a washing step follows in which the sample is washed with distilled or deionized water. The washing step can be repeated for several times. Alternatively, mild acids can be used for washing the sample and for utilizing residual alkaline. In preferred embodiments, the washing step is carried out with 0.1 N acidic acid or citric acid.

The method further comprises a cleaning step (denaturing step) with a chaotropic agent. In this step, non-collageneous components are denatured, whilst the collagen matrix is maintained. The impurities are thus removed from the pores within the collagen matrix. In a preferred embodiment of the invention, the chaotropic agent is a guanidinium salt, a perchlorate, urea, MgCl₂, a thiocyanate, an iodide or a barium salt.

Chaotropic agents are substances which disrupt the three-dimensional structure of macromolecules, such as proteins, DNA or RNA, and thus denature them. Chaotropic agents are known to interfere with stabilizing intramolecular interactions mediated by non-covalent forces such as hydrogen bonds, van der Waals forces and hydrophobic effects. Such substances are well-known to the skilled person. They are classified in the Hofmeister series in the order of their ability to change the water structure. Especially preferred is the use of guanidinium chloride. Guanidinium is used in biochemistry as a means of extraction. Specifically, it is known to denature proteins. Highly concentrated guanidinium chloride is used in the art for removing proteoglycans from tissue. In the present invention, the chaotropic agent efficiently extracts proteoglycans. In contrast, the collagen matrix is not denatured or negatively affected. Guanidinium chloride at high concentrations induces swelling of the tissue and thus facilitates the extraction of impurities.

In a preferred embodiment, guanidinium chloride is used at a relatively low concentration between 0.2 and 3 M, preferably between 0.5 and 2.5 M, especially preferred 1 M. It was found that a concentration of guanidinium chloride of 4 M or higher, which is used in the art, can result in less efficient extraction of contaminants.

In a preferred embodiment, the cleaning step is applied for at least 6 hours, more preferably for at least 1 or 2 days. It was found that especially when the sample is a thick tissue such as meniscus, efficient diffusion of the chaotropic agent into the core of the tissue is possible during such an extended time. Preferably, the chaotropic agent is used in an aqueous solution, for instance an appropriate buffer. In a preferred embodiment, proteinase inhibitors are added, because proteases can be released by the tissue during removal of proteoglycans and subsequent exposure of internal collagen.

Preferably, at the end or during the cleaning step, the sample is subjected to a sonication. At the end of the cleaning step, the chaotropic agent solution is removed from the sample. Subsequently, preferably one or more washing steps are applied, preferably with distilled or deionized water. Preferably, during the washing steps, further sonication steps are applied.

Preferably, the cleaning step is carried out in the presence of sodium acetate, preferably at a concentration between 0.01 and 0.1 M. A preferred temperature range is from 0 to 25°C, more preferably between 0 and 8°C.

The treatment with the chaotropic agent allows efficient removal of proteoglycans. During removal of the proteoglycans, it is possible that proteases are released which are inhibited by the chaotropic agent. This effect is especially pronounced when the cleaning step is carried out in a buffer, for instance a sodium acetate buffer.

In a preferred embodiment of the invention, the method further comprises at least one defatting step in a solution which is less polar than water, or in a detergent solution. In general, a solvent or detergent is selected which partially or fully dissolves and removes fats, lipids and other lipophilic components from the sample. In a preferred embodiment of the invention, the solvent is selected from methanol, ethanol, propanol, isopropanol, butanol, isobutanol, acetone or a mixture of any of these with water. In a preferred embodiment, a 20 to 90% solution of ethanol is used.

Alternatively or in an additional step, a solution of a detergent in water can be used. A preferred detergent is Triton X-100. Detergents such as SDS, which bind to collagen, are less preferred.

Preferably, the defatting step is carried out for a period between 20 minutes and 48 hours. A preferred temperature range is between 0°C and 40°C, preferably at room temperature.

In the method of the invention, it is preferred to carry out the defatting step after the alkaline treatment. This is advantageous, because the alkaline treatment step induces swelling of the tissue, removes non-collageneous matrix components, and the defatting is more efficient at the inside of the tissue.

In a preferred embodiment of the invention, the method further comprises at least one inactivation step with an aqueous solution of a peroxide. In preferred embodiments, the peroxide is hydrogen peroxide, an inorganic peroxide salt or an organic hydroperoxide. Useful inorganic peroxide salts are monovalent or bivalent salts ofNa, K, Ca, Sr, Ba, Li and Mg. Useful organic peroxides are ether or ester peroxides.

In a preferred embodiment of the invention, the peroxide is hydrogen peroxide. Preferred is a concentration range between 2 and 25% by weight, more preferred between 3 and 10% by weight. The peroxide may be used in combination with a buffer or a salt, such as K⁺, NH⁺, Cl⁻, SO₄²⁻ ,CO₃²⁻ or hydrogen phosphate. The salt can be used in a concentration between 0,5 and 10, or between 1 and 5% by weight. The amount of peroxide is chosen such that the matrix is essentially not decomposed.

Preferably, the peroxide inactivation is applied for 1 to 48 or 1 to 24 hours, preferably for at least 2 or at least 5 hours, preferably in a darkened environment. The conditions can be adjusted such that the peroxide solution is exchanged after decomposition of the peroxide. A preferred temperature range is between 0 and 40°C, more preferably between 4 and 30°C. At the end of the peroxide treatment, it is preferred to apply a sonication. Subsequently, the peroxide solution is removed from the sample, and the sample is subjected to at least one washing step. Preferably, the washing steps are supported by sonication. The washing steps are preferably carried out in distilled or deionized water.

In a preferred embodiment of the invention, the method further comprises at least one washing step after at least one treatment step and/or prior to the first treatment step. In general, after each of the specific purification steps of the method of the invention, i.e. the alkaline treatment, defatting step, the cleaning and the inactivation step, the sample is removed from the specific purification solution, and one or more washing steps can be applied. Surprisingly, it was found that in contrast to procedures known in the art, the washing steps in the inventive method can be carried out with water, preferably distilled water or deionized water. In contrast to known procedures, it is not necessary to use buffers in which physiological conditions have been adjusted. The inventive method is thus simplified. However, the washing steps in the inventive method can also be carried out with buffers under physiological conditions. The washing buffers may also comprise further additives, such as protease inhibitors or buffer salts. Each washing step may be repeated several times, for instance 2 to 10 times, especially 2 or 4 times. The washing steps may be applied for 10 minutes up to 48 hours, preferably for 30 minutes up to 5 hours. Here in the washing steps, sonication treatments can be applied in order to enhance the washing effect. For instance, a sonication can be carried out for 2 to 30 minutes, preferably for 5 to 50 minutes.

In a preferred embodiment of the invention, the method comprises the steps of:
(a) at least one alkaline treatment in an alkaline solution at a pH >12,
(b) at least one defatting step with at least one solvent which is less polar than water, or with a detergent solution,
(c) at least one cleaning step with at least one chaotropic agent and
(d) at least one inactivation step with an aqueous solution of a peroxide.

In a preferred embodiment, the steps are carried out in the order from (a) to (d). Depending on the specific tissue, steps (a) to (d) can also be applied in a different order. For instance, the inactivation step (d) can be applied after the defatting step (b) and/or the cleaning step (c). It is preferred that the treatment of the tissue starts with step (a).

Any of the treatment steps (a) to (d) can be repeated, for instance applied 2 or 3 times. Specifically, it is preferred that the peroxide inactivation step is repeated at least once.

In a preferred embodiment of the invention, the method comprises, preferably in the following order, the steps of:
(a) at least one alkaline treatment in an alkaline solution at a pH >12.5,
(b) at least one defatting step in a solvent which is less polar than water,
(c) at least one cleaning step with a guanidinium salt and
(d) at least one inactivation step with an aqueous solution of hydrogen peroxide.

In a preferred embodiment of the invention, each of steps (a) to (d) is following by at least one washing step and/or wherein at least one washing step is carried out prior to step (a).

In a preferred embodiment of the invention, at last one washing step is carried out in distilled or deionized water.

In a preferred embodiment, prior to the alkaline treatment step (a), the sample is thoroughly washed. For instance, the washing of the tissue can be carried out with deionized/distilled water at a temperature between 0 and 40°C, preferably 4 to 30°C. The washing step may be carried out for 12 to 72 hours. During this time, the water is preferably replaced by fresh water several times.

After the final purification step, for instance after the last inactivation step (d) with a solution of a peroxide, the sample, which is a highly pure collagen tissue, is preferably washed thoroughly. For instance, the washing step can be carried out with water for up to 72 hours at a temperature between 0 and 40°C, preferably between 4 and 30°C. The water may be replaced several times.

The sample thus purified can be adapted for storing and stored for extended times. For instance, the purified sample can be stored in sodium chloride solution, for example in a concentration range between 0.1 and 26 percent by weight. The sample may be stored sterile under vacuum or irradiated, for instance by UV and gamma-rays. Alternatively, the sample may be stored at temperatures below - 10°C or below -18°C.

In a preferred embodiment, the purification steps of the invention and the washing steps or any other treatment steps are carried out under a steady movement of the solutions and the sample. Useful devices are known in the art and comprise various laboratory shakers, such as overhead shakers, horizontal shakers, spinning shakers, possibly supported by pressure or suction forces, by vortexing or by sonication. The treatment steps may be carried out in a reaction chamber (washing reactor).

In addition to the purification steps (a) to (d) outlined above, it is possible to carry out further purification steps known in the art. For instance, it is possible to carry out an additional acid treatment step (e). Such an acid treatment step is used for instance in the US 5,993,844. It was found that the method disclosed therein is not efficient in purifying a thick tissue, such as a meniscus tissue. An efficient removal of further components from the collagen matrix is only observed according to the invention with a strong alkaline treatment step and a cleaning step with a chaotropic agent. However, an additional treatment with an acidic solution, for instance at a pH between 0 and 1, or at a pH between 0 and 8, is possible. The additional acid treatment step (e) may be carried out after the alkaline treatment step (a). In a specific embodiment of the invention, the method does not comprise an acid treatment step.

In a preferred embodiment of the invention, the method does not comprise an enzyme treatment, such as a proteolytic enzyme digestion step. Such a step is known from US 5,374,539. Since the present invention efficiently removes the contaminants from the collagen matrix in the sample, such an enzyme treatment step is not necessary. The method is thus simpler, cheaper and more reliant. In a less preferred embodiment of the invention, an additional proteolytic enzyme digestion step is applied. In another embodiment of the invention, proteolytic enzymes may be added during any of the treatment steps (a) to (g).

In the US 5,993,844, the alkaline treatment step is carried out in the presence of a chelating agent. According to the present invention, it is not necessary to apply the alkaline treatment in the presence of a chelating agent. In an embodiment, no chelating agent is added in step (a). However, a chelating agent may be added in the method of the present invention in the alkaline treatment step (a) or in another step if considered advantageous.

As outlined above, the inventive method can be carried out at standard temperatures and with standard chemicals, which are cheap, easily available and reliable. The steps comprise simple procedures such as addition and removal of liquids, incubation and sonication. It is not necessary to treat the tissue individually, by cutting, shaping, pressing etc. The inventive method can thus be applied in an automated process. This is highly advantageous in the routine preparation of a large number of implants.

Another subject of the invention is a highly pure collagen obtainable by a process of the invention. Preferably, the highly pure collagen is a meniscus collagen.

Preferably, "highly pure" means that the collagen comprises more than 95, 98, 99 % or 99,5 (of total dry weight) collagen. The degree of non-collageneous impurities, such as proteoglycans, glycosaminoglycans (e.g. hyaluronic acid) and cells, is thus low. Such impurities are potential antigens. Preferably, the amount of cells or proteoglycans in the inventive highly pure collagen is less than 0.5% or less than 0.1% of the level in the original tissue, or not detectable at all. It was found that in a highly pure collagen of the invention, the amount of cells can be reduced so much that cell nuclei are not detectable any more by staining.

In a preferred embodiment, the content of proteoglycans in the inventive collagen implant is below 0,5 or below 0,2 percent of total dry weight.

Preferably, the thickness of the sample and/or the inventive highly pure collagen is at least 1, 3 or 5 mm, preferably between 1 mm and 50 mm, more preferably between 3 and 25 mm. The dimensions of the sample and/or inventive highly pure collagen may be from 0.5 to 50 cm².

Another subject of the invention is an implant comprising a highly pure collagen of the invention. The collagen may be used directly as an implant. The highly pure collagen may also be adapted to an appropriate size and/or adapted further to use as an implant, for instance by sterilization or by equipping the collagen with agents such as pharmaceutics.

Another subject of the invention is the use of the highly pure collagen of the invention, or a piece thereof, as a collagen implant or in the preparation of a collagen implant. The implant can be used for allogenic or xenogenic transplantation. Another subject of the invention is a surgical method, in which an implant of the invention is transferred to a patient in need thereof or to a non-human animal, such as a horse or dog. The implant obtained according to the process is highly sterile and the simple process steps avoid cross-contaminations.

The highly pure collagen of the invention can also be used as a scaffold for cell-based tissue engineering, preferably for meniscus or cartilage. The scaffold is preferably chondroconductive and chondroinductive. Thereby an implant can be prepared by ex vivo modification of the collagen.

The method and the collagen of the invention solve the problem underlying the invention. The method of the invention provides a relatively simple and efficient way for obtaining highly purified collagen for use as an implant at low costs. The inventive method preserves the three-dimensional structure of native collagen. The inventive method removes or inactivates all additional components of native tissue except the collagen. The inventive collagen implant thus does not induce undesired immune responses and rejection of implants. The highly purified collagen matrix allows diffusion of cells into the matrix and growth of new tissue after implantation. Due to the specific purification steps, the inventive collagen is highly sterile. Preferably, the implant does not comprise active pathogens, such as fungi, viruses, bacteria, prions and/or spores. It is not necessary to apply additional sterilization steps to the inventive collagen. The inventive implant is especially useful for patients who are oversensitive against chondroitin sulfate.

The inventive collagen matrix is chondroconductive. After implantation, the collagen adapts chondroinductive properties, which are induced by diffusion of cells of the host into the matrix.

The inventive method can be applied to any natural collagen sample. The inventive method is especially useful for dense tissues, such as meniscus. Methods known in the art commonly do not provide purification of the matrix and efficient removal and inactivation of other components from the interior of the matrix. The inventive implant allows purification or a large piece of meniscus whilst maintaining the natural collagen matrix. The outer form, size and geometry of the meniscus are maintained. The implant is useful in total or partial meniscus ectomy, because according to the inventive method it is possible to prepare a full natural meniscus or any part thereof. The purified meniscus of the invention can be attached to the synovial membrane.

Since the present invention allows purification and inactivation throughout a dense collagen matrix, whilst maintaining the collagen structure, it is not necessary to disrupt the matrix prior to purification and/or to cross-link the matrix after the purification, as in the US 5,374,539. The present inventive method is simpler and more efficient compared to a method which requires disruption of the matrix, addition of enzymes and cross-linking of the matrix.

The inventive meniscus which preserves the natural structure is also significantly distinct form implants known in the art, in which collagen of type I only is purified, crosslinked and pressed into a required form. The inventive purified meniscus already is available in a natural meniscus form.

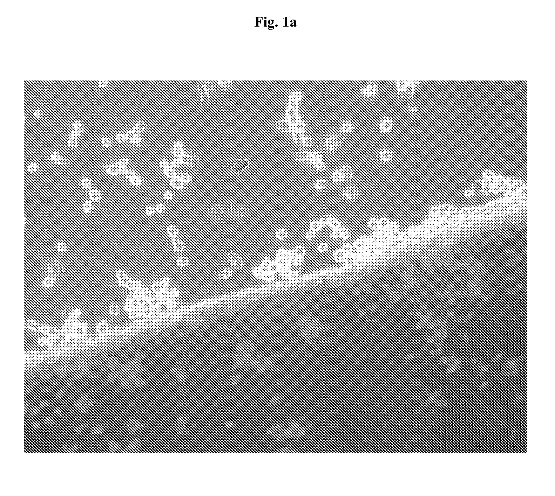

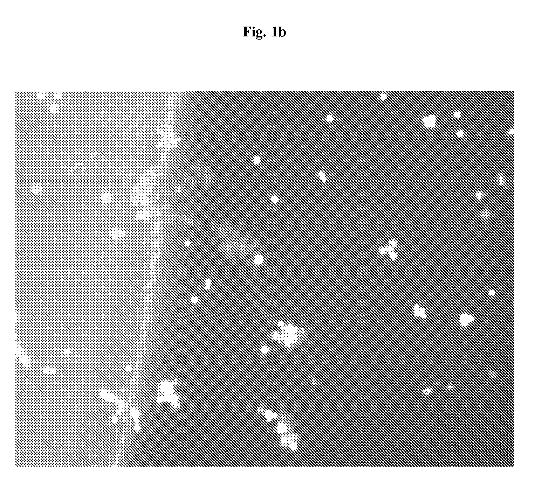

Figures 1a and 1b show CFDA SE/PI vital staining of 3T6-fibroblast on meniscus graft. Vital cells show green fluorescence (circular, white or light grey) whereas dead cells fluoresce red. Vital 3T6 fibroblasts adhere on the surface of meniscus graft.

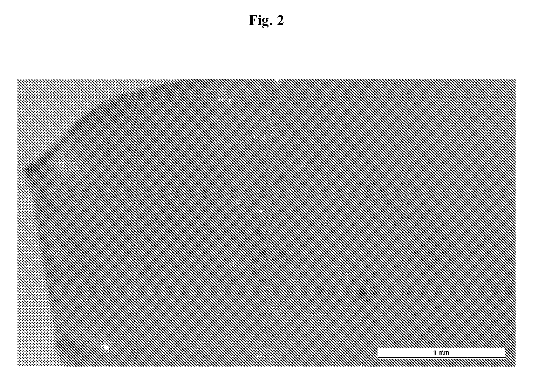

Figure 2 shows dark violet-stained stained (circular, dark grey/black) vital 3T6 fibroblast cells in the matrix of a 0.75 mm thick tissue section along the distal edge of the meniscus. Figures 1 and 2 show that the meniscus graft is not cell toxic.
   Growing up cells are vital and adhere.

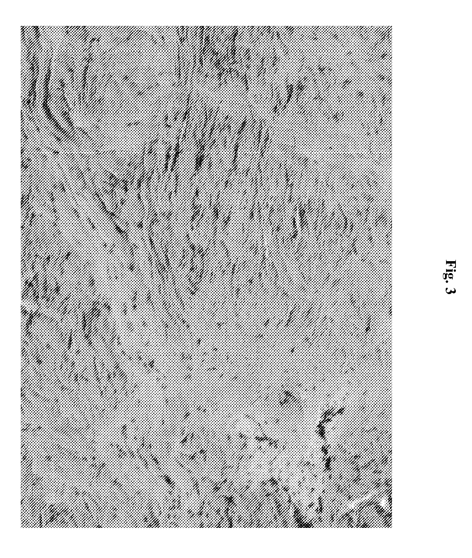

Figure 3 shows haematoxylin-eosine (HE) stained cell nuclei in the cryo-cut section of a native meniscus. Cell nuclei are black stained. The extracellular matrix is stained red (light grey).

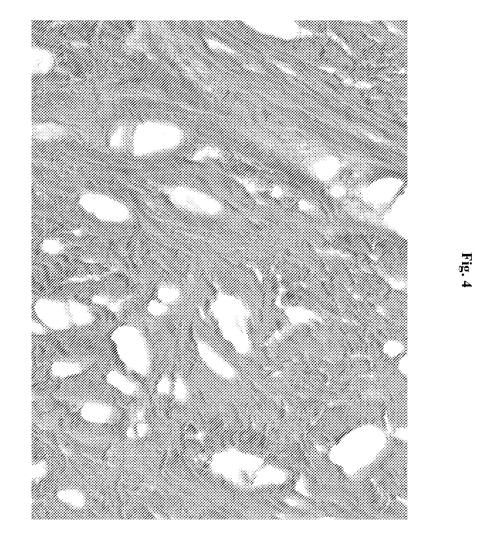

Figure 4 shows haematoxylin-eosine (HE) staining of a cryo-cut section of a processed meniscus. The structure is loosed by the removal of extracellular matrix and cutting. Cell nuclei (circular, black) cannot be detected. The comparison of figures 3 and 4 thus show the effectiveness of cell removal

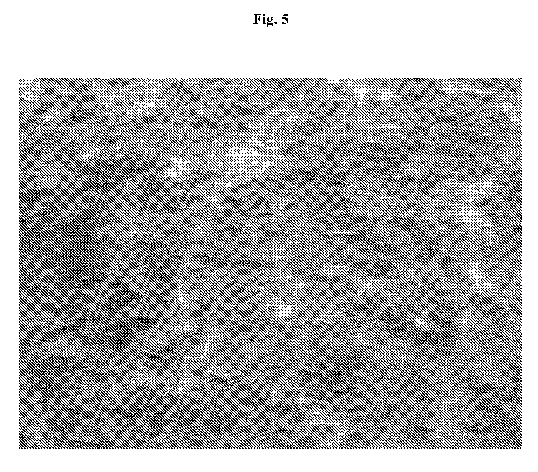

Figure 5 shows alcian blue staining of glycosaminoglycans in the cryo-cut section of a native meniscus. Especially sulfated and acid glycosaminoglycans, e.g. chondroitin and kreatan sulfate, are stained dark blue (dark grey). Cell nuclei remain undyed.

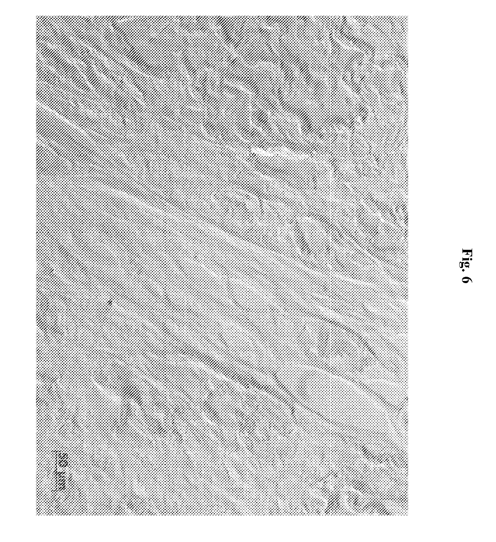

Figure 6 shows alcian blue staining of glycosaminoglycans in the cryo-cut section of a processed meniscus. Glycosaminoglycans cannot be detected. The comparison of figures 5 and 6 thus shows the effectiveness of the removal of glycosaminoglycans.

### Examples:

In the following example, the inventive method is applied to a meniscus tissue.

### 1. Pretreatment - Washing

The tissues are processed in 0.8 L H2O(deion) for 24 h in a 1 L plastic bottle. During the first 10 h every 2 hours the tissue is treated 10 min in an ultrasonic bath. After every ultrasonics treatment the water is exchanged for fresh H₂O(deion).

### 2. Alkaline treatment for cleaning and inactivation

After 24 h, directly after the separation of the water the tissues are transferred into 0.5 L of 1 N NaOH solution and processed for 3 h. At the end follows 10 min of an ultrasonic application. The NaOH solution is separated.

### 3. Washing step

The tissues are washed 24 h with 0.8 L H₂O(deion) for the removal of NaOH solution and hydrolysis products. During the first 4 h every 30 min the water is changed. The pH value of the washing solution should be controlled. If necessary, washing must be continued.

### 4. Defatting step

After separation of the washing solution the tissues are treated in 0.5 L of 70 % ethanol for 3 h. At the end follow 10 min of an ultrasonic application.

### 5. Washing step

After separation of the aqueous ethanol extract the tissues are washed with 0.5 L H₂O(deion) for 1 d. After 2 and 4 h an ultrasonic application for 10 minutes follows with a change of the washing solution. Afterwards the water will be changed 2 times more and separated at the end.

### 6. Denaturation step

The tissues are transferred into 0.5 L glass bottles and chemically treated in 0.25 L of 1 M Guanidine-HCl solution and 0.05 M sodium acetate in incubation buffer at 4 °C on an horizontal shaker. At the 2nd and 4th day follow 10 min of ultrasonic treatments. At the end the reaction solution is separated.

### 7. Washing step

The tissues are washed in 0.5 L H₂O (deion) for 24 h. After 2 and 5 h follow 10 min ultrasonic treatments, whereas the washing water is changed after each treatment. At the end the washing water is separated.

### 8. Additional inactivation and cleaning step

The tissues are treated at 4 °C, in the dark, for 24 h in 0.5 L sterile filtrated, preferably 3-5 % H₂O₂ solution in sterile bottles on an horizontal shaker. At the end follow 10 min ultrasonic treatment. Afterwards the H₂O₂ solution is separated.

### 9. Washing step

The tissues are cleaned for 24 h in 0.5 L sterile H₂O(deion) During the first 3 h every hour the tissues are treated with ultrasonic for 10 min. Every time the water is changed.

### 10. Storage

The meniscal graft should be stored appropriate for the specific surgical operation. The tissue can be, among others, sterile air dried or dehydrated in a series of ascending concentrations of acetone or other solvents, sterile, vacuum-packed stored, and / or in 0.9 to typical 26 % NaCl solution conserved and / or frozen at minimum -18 °C. At the end the tissue can be rayed by UV light or gamma-rays.

### General Comments:

The use of an overhead shaker performed the best results with easiest usage. Other mixing and shaking processes lead to similar qualities.

Optional ultrasonic applications, e.g. in an ultrasonic bath, at every procedural step lead for most, but not for all meniscus to a better quality. Number and duration of the applications are to be adapted to thickness and size of the tissue. The given times are standard values and can be adapted to the tissue and the general conditions.

The choice of bottles, jars or vessels (hereinafter called bottles) of inert materials is preliminary determined from the size of the shaker. This description of the operating procedure does not regulate, and is not restricted by the kind of inert material such as glass or plastics. With respect to the movement of the meniscus body in the bottle with minimal contacts with the wall, a bottle with higher volume has advantage over smaller ones. To minimize costs the volume can be reduced at the costly steps, it should preferably be at least 0.5 L. The bottle should preferably be filled at least 50 %. The specified volumes refer to one meniscus body. Because of the probability of a contact each other, and consequently abrasion, only one meniscus body per bottle should be processed. The process can be conducted in a reactor.

The number of washing steps and the exchanges of the solutions have to be adapted to the thickness and size of the tissue. The process time for single steps can be changed. This extends mainly the overall process time.

If not otherwise declared, all steps, with the exception of the ultrasonic application, are processed in an overhead shaker, and at room temperature.

The process should preferably be carried out under germ-free, sterile conditions in sterile bottles. At least with the inactivation step with H₂O₂ solution sterile conditions should be kept.

In general, the optimum temperature range is 20 °C - 25 °C. Principally, the temperature should lie in the range of 0 °C to 35 °C. Low temperature decreases, amongst others, kinetics and extends the process time, and higher temperature increases, among others, the risk for collagen denaturation and microbial growth.

### Remarks for step 1

The meniscus should be cleaned at least externally from soluble substances. These can be salt solutions from storage as well as residues. Additionally, for advanced pretreatment, an organic solvent, pure or as an aqueous mixture, such as an alcohol, can be used to remove external adhering lipophilic substances.

### Remarks for step 2

Alternatively, solutions with KOH, and other alkaline active substances can be used. The NaOH solutions preferably should be at least 0.2 N and no more than 3 N. The process time for the treatment with NaOH solution preferably is preferably 2.5 to 3.5 h. Within this time the solution should penetrate into the centre of the tissue. The efficiency of diffusion depends on the nature and size of the sample. Diffusion experiments show that in some meniscus even with a 3 N NaOH solution the centre is not reached until 2.5 h. As from approximately 3.5 h the higher cleaning efficiency should be considered against the increasing collagen denaturation.

### Remarks for step 3

Alternatively, weak acids such as 0.1 N acetic and citric acid can be used. In case of acid excess the tissue is swelling again, and the reaction washing time should be accordingly adapted. The amount of acid and the acid concentration are correct, if the tissue swelling is reduced and remains constant. The pH value should be controlled carefully. After longer reaction time the fraction of denatured collagen increases. Typically, the time has to be reduced to 3 h.

### Remarks for step 4

The aqueous solutions can be prepared with other organic solvents such as acetone, isopropanol and similar ones with comparable polarity and hydrophobicity, too. The water content preferably lies in the range of 10 % to 50 %. Alternatively or additionally detergents like Triton X-100 can be used.

### Remarks for step 6

Composition of the incubation buffer: 0.1 M Tris-HCl, 1.0 mM iodoacetamide, 1.0 mM EDTA, 10 µg/L pepstatin A. Other buffer systems can be used, preferably with protease inhibitors. The substitution by water can lead to a reduced extraction efficiency. Apart from Guanidine-HCl other chaotropic salts such as MgCl₂ or urea can be used with or without sodium acetate resp. alternative salts. The volume can be increased but is kept small for economic reasons. The optimum ratio is preferably 15 to 20 of the tissue. Smaller bottles resp. smaller volumes may increase abrasion.

### Remarks for step 8

This step increases the security for an inactivation of the bioimplant. Before this step an additional alkaline treatment step with subsequent washing can be added. This is recommendable especially if the rinsing solutions do not penetrate completely into the depth of the tissue. At least from here on working under sterile conditions is recommended.

### Remarks for step 9

If the inactivation step is omitted this washing step can be skipped, too.

### Literature:

Delloye, C., O. Cornu, et al. (2007). "Bone allografts - what they can offer and what they cannot." J Bone Joint Surg [Br] 89-B: 574-9.

Goble EM, V. R. (1999). "Meniscal substitutes - human experience." Scand J Med Sci Sports(9): 146-157.

Goncalves AC, Griffiths LG, Anthony RV, Orton EC. (2005). "Decellularization of bovine pericardium for tissue-engineering by targeted removal of xenoantigens." J Heart Valve Dis 14:212-217.

Rodeo, S. A., et al. (2000). "Histological analysis of human meniscal allografts: a preliminary report." The Journal of Bone and Joint Surgery 82: 1071-1082.

Verdonk, R. (1997). "Alternative treatments for meniscal injuries." The Journal Of Bone and Joint surgery 79-B(5): 866-873.

Wirth, C. L., G. Peters (2002). "Long-term Results of Meniscal Allograft Transplantation." American Journal of Sports Medicine 30(2): 174-181.

## Claims

1. A method for preparing a highly pure collagen, comprising subjecting a sample comprising collagen to
at least one alkaline treatment step in an alkaline solution at a pH >12 and
at least one cleaning step with a solution of at least one chaotropic agent.

2. The method of claim 1, wherein the sample comprises a three-dimensional collagen matrix.

3. The method of any of the preceding claims, wherein the sample is selected from meniscus, intervertebral (or spinal) discs, articular discs, ear cartilage, rib cartilage, rib cartilage, nasal cartilage, cruciate ligament and Achilles tendon.

4. The method of any of the preceding claims, wherein the alkaline solution comprises NaOH, KaOH, Ca(OH)₂, CaO, Mg(OH)₂ or LiOH.

5. The method of any of the preceding claims, wherein the chaotropic agent is a guanidinium salt, a perchlorate, urea, MgCl₂, a thiocyanate, an iodide or a barium salt.

6. The method of any of the preceding claims, further comprising at least one defatting step with at least one solvent which is less polar than water, or with a detergent solution.

7. The method of claim 6, wherein the solvent is methanol, ethanol, propanol, isopropanol, butanol, isobutanol, acetone or a mixture of any of these with water, or wherein the detergent is Triton X-100.

8. The method of any of the preceding claims, further comprising at least one inactivation step with an aqueous solution of a peroxide, preferably hydrogen peroxide.

9. The method of any of the preceding claims, further comprising at least one washing step after at least one step and/or before the first step.

10. The method of any of the preceding claims, wherein the method comprises the steps of:
(a) at least one alkaline treatment step in an alkaline solution at a pH >12,
(b) at least one defatting step with at least one solvent which is less polar than water, or with a detergent solution,
(c) at least one cleaning step with a solution comprising at least one chaotropic agent and
(d) at least one inactivation step with an aqueous solution of a peroxide.

11. The method of any of the preceding claims, wherein each of steps (a) to (d) is followed by at least one washing step and/or wherein at least one washing step is applied before step (a).

12. A highly pure collagen obtainable by a process of any of the preceding claims.

13. A highly pure collagen implant comprising more than 99 percent (dry weight) collagen.

14. A collagen implant comprising a highly pure collagen of claim 12 or 13.

15. The use of the highly pure collagen of claim 12 or 13, or a part thereof, as a collagen implant or for the preparation of a collagen implant.
